# EUROPEAN PATENT SPECIFICATION

(11) **EP 1 711 482 B1**
(45) Date of publication and mention of the grant of the patent: **04.07.2007**
(21) Application number: 05707936.0
(22) Date of filing: 03.02.2005
(51) Int. Cl.: C07D 301/12

(54) **PROCESS FOR PREPARING ALKYLENE OXIDE**
VERFAHREN ZUR HERSTELLUNG VON ALKYLENOXID
PROCEDE DE PREPARATION D'OXYDE D'ALKYLENE

(30) Priority: 05.02.2004 EP 04250625
(43) Date of publication of application: 18.10.2006
(73) Proprietor: SHELL INTERNATIONALE RESEARCH MAATSCHAPPIJ B.V., 2596 HR Den Haag (NL)
(72) Inventor: DU CAUZE DE NAZELLE, Gerard, Jurong Island 627879 (SG)
(74) Representative: Zeestraten, Albertus W. J.
(86) International application number: PCT/EP2005/050472
(87) International publication number: WO 2005/075444

(56) References cited:
- EP-A- 1 209 155
- EP-A- 1 243 585
- EP-A- 1 266 895
- WO-A-02/090340
- US-A1- 2003 125 574
- US-A1- 2003 158 447
- US-A1- 2003 191 327
- US-B1- 6 583 300

## Description

The present invention relates to a process for the preparation of an alkylene oxide.

### Background of the invention

Processes for preparing propylene oxide employing organic hydroperoxides, are well known in the art. As described in US-A-5,883,268, such process conventionally comprises peroxidation of ethylbenzene, followed by contacting the peroxidation reaction product with aqueous base in amount sufficient to neutralize acidic components thereof and separating the resulting mixture into an aqueous stream and a deacidified organic stream. The base contaminated, deacidified hydroperoxide stream is washed with water. The product obtained can be used in the catalytic epoxidation of propene to form propylene oxide using a solid heterogeneous titanium containing catalyst.

Although the epoxidation reaction is exothermic, the temperature of the mixture of hydroperoxide and alkene is usually increased before use in the epoxidation process. This ensures best use of the catalyst employed. However, it was found that the heat exchanger used for increasing the temperature of the mixture, fouled quickly when contacted with the mixture of hydroperoxide and alkene. It is disadvantageous if the temperature of the mixture of hydroperoxide and alkene cannot be increased as this makes that deactivation of the catalyst cannot be compensated for. This results in more frequent replacement of the epoxidation catalyst.

### Summary of the invention

It has now been found that the temperature of the reaction mixture to be contacted with the epoxidation catalyst can be heated to relatively high temperature if a specific set-up is used. In this specific set-up the temperature of the mixture of alkene and hydroperoxide is increased by increasing the temperature of the alkene only. Heating the alkene to a relatively high temperature did not cause fouling of the heat exchanger. Without wishing to be bound to any theory, it is thought that the fouling of the heat exchangers when heating a mixture of alkene and a hydroperoxide stream is caused by salts which are present in the hydroperoxide stream. The salts seem to deposit on the surface of the heat exchanger when the hydroperoxide stream is mixed with the alkene.

Surprisingly, it has now been found that the reaction mixture for the epoxidation reaction could be brought to relatively high temperature without substantial fouling of the heat exchanger if the alkene only was increased in temperature.

Therefore, the present invention relates to a process for the preparation of alkylene oxide which process comprises:
(a) oxidation of an organic compound to obtain a hydroperoxide containing stream,
(b) washing the hydroperoxide stream with a basic aqueous solution,
(c) washing the hydroperoxide stream of step (b) with water,
(d) optionally subjecting the hydroperoxide stream obtained in step (c) to distillation,
(e) contacting at least part of the hydroperoxide stream obtained in step (c) and/or (d) with alkene and a heterogeneous catalyst to obtain a reaction mixture containing a hydroxyl containing compound and alkylene oxide, and
(f) separating at least part of the alkylene oxide from the reaction mixture,
in which process the alkene added in step (e) has a temperature of from 60 to 120 °C while the temperature of the hydroperoxide stream contacted with the alkene is similar to the temperature of the hydroperoxide stream obtained in step (c) and/or (d).

### Detailed discussion of the invention

In step (a) of the present invention, an organic compound is oxidised. The organic compound preferably is an alkyl substituted aromatic compound. Thecompounds which are most preferably used in the process of the present invention are benzene compounds containing at least 1 alkyl substituent which alkyl substituent contains of from 1 to 10 carbon atoms, preferably of from 2 to 8 carbon atoms. Preferably, the benzene compound contains on average of from 1 to 2 constituents. The alkylaryl compounds most frequently encountered are ethylbenzene and cumene. In that case, the hydroperoxide compounds formed are ethylbenzene hydroperoxide and cumene hydroperoxide.

The oxidation of the alkylaryl compound can be carried out by any suitable process known in the art. The oxidation can be carried out in the liquid phase in the presence of a diluent. This diluent is preferably a compound which is liquid under the reaction conditions and does not react with the starting materials and product obtained. However, the diluent can also be a compound necessarily present during the reaction. For example, if the alkylaryl is ethylbenzene the diluent can be ethylbenzene as well and if the alkylaryl is cumene the diluent can be cumene as well.

Besides the desired hydroperoxide compound, a range of contaminants are created during the oxidation of organic compounds. Although most of these are present in small amounts, it has been found that the presence of compounds such as organic acids can cause problems in further use of the hydroperoxide containing stream. As described in US-A-5,883,268, the conventional method of reducing the amount of contaminants is by contacting the hydroperoxide stream with a basic aqueous solution.

Aqueous base most often used in conventional processes, are sodium and/or potassium containing bases such as sodium hydroxide, potassium hydroxide, sodium carbonate and potassium carbonate. Most preferably, the basic aqueous solution used in the present invention is an aqueous solution of sodium hydroxide. Preferably, the washing with a basic aqueous solution is carried out at a temperature of between 0 °C and 150 °C, more preferably of between 20 °C and 100 °C.

The washing of step (b) comprises both the contact with the basic aqueous solution and the separation into a hydrocarbonaceous phase and an aqueous phase. A preferred separation method comprises allowing the hydrocarbonaceous phase and aqueous phase to settle in a settling vessel and subsequently separating a hydrocarbonaceous phase from an aqueous phase. The hydrocarbonaceous phase containing hydroperoxide can subsequently be sent to a coalescer where further aqueous phase is removed. Preferably, the separation step is carried out at a temperature of between 0 °C and 150 °C, more preferably of between 20 °C and 100 °C.

The hydroperoxide containing stream which has been treated with basic aqueous solution can be contacted with alkene without further treatment. However, contact with the basic aqueous solution introduces alkali metal into the hydroperoxide containing reaction product. Therefore, the hydroperoxide stream is subsequently washed with water.

The water wash of step (c) can be carried out in any way known to someone skilled in the art. The water which can be used can contain contaminants, such as organic compounds. Such contaminants may have been introduced by the recycle of at least part of the wash water, either to the same wash step or to another wash step. The water can be fresh water only, it can be a combination of fresh water containing substantially no contaminants and one or more different waste water streams, or it can consist only of different kinds of waste water streams or it can consist of a single type of waste water.

The exact conditions under which the water wash is carried out, strongly depend on further circumstances. Preferably, the water wash is carried out at a temperature of between 0 °C and 150 °C, more preferably of between 20 °C and 100 °C. The hydrocarbonaceous phase and the aqueous phase subsequently can be carried out in any way known to someone skilled in the art. A preferred separation method comprises allowing the hydrocarbonaceous phase and aqueous phase to settle in a settling vessel and subsequently separating a hydrocarbonaceous phase from an aqueous phase. The hydrocarbonaceous phase containing hydroperoxide can subsequently be sent to a coalescer where further aqueous phase is removed. Preferably, the separation step is carried out at a temperature of between 0 °C and 150 °C, more preferably of between 20 °C and 100 °C.

The water wash can be carried out once or it can be repeated several times.

The reaction product of step (c) can be sent to step (e) as such. However, it is preferred to remove light compounds. Such light compounds can be unconverted organic compounds, water and contaminants. The light components can be removed easily by subjecting the reaction product of step (c) to distillation, preferably distillation at reduced pressure. A distillation which is especially suitable is so-called flash distillation, which comprises distillation at very low pressure. It has been found that such flash distillation is efficient in removing light compounds such as oxygen and light acids formed during the oxidation.

In process step (e), at least part of the hydroperoxide stream obtained in step (c) and/or (d) is contacted with alkene in the presence of a heterogeneous catalyst to obtain a reaction mixture containing a hydroxyl containing compound and alkylene oxide. The alkene preferably is propene which makes that the alkylene oxide obtained is propylene oxide.

The hydroperoxide stream added to step (e) has a temperature which is similar to the temperature of the hydroperoxide stream obtained in step (c) and/or (d). Although it can sometimes be advantageous to slightly increase the temperature of the hydroperoxide stream, for example if the stream is obtained directly from step (c), it is preferred that the temperature of the hydroperoxide stream should be increased not more than 10 °C, more specifically not more than 5 °C. Preferably, the temperature is not increased.

The alkene added in step (e) has a temperature of from 60 to 120 °C before contact with the hydroperoxide stream. This desired temperature usually is obtained by heating the alkene. The alkene generally has a temperature of from 40 to less than 60 °C before heating, more specifically of from 45 to 55 °C. Usually, the alkene will be heated with the help of a heat exchanger. The stream against which the heat is exchanged can be any stream having the right temperature. Generally, steam will be used for heating the alkene. Preferably, the alkene to be introduced in step (e) has a temperature of at least 70 °C, more preferably more than 75 °C, more preferably more than 80 °C. The alkene preferably has a temperature of at most 115 °C, more specifically at most 110°C, more specifically at most 105 °C, most. specifically at most 100 °C. Preferably, the temperature of the alkene added in step (e) is of from 70 to 110 °C before contact with the hydroperoxide stream.

The specific temperature of the hydroperoxide stream and of the alkene depend on the exact circumstances such as amount of catalyst available for the reaction, the activity of the catalyst in general, the degree to which the catalyst has become deactivated and the molar ratio of alkene to hydroperoxide.

Generally, the hydroperoxide stream added in step (e) will have a temperature of from 70 to 120 °C before contact with the alkene. More specifically, the temperature of the hydroperoxide stream added in step (e) generally will have a temperature of from 80 to 110 °C. Preferably, the temperature of the hydroperoxide stream is at least 85 °C, more specifically at least 90 °C. The temperature of the hydroperoxide stream preferably is at most 105 °C, most preferably at most 100 °C.

As mentioned above, an important advantage of the process of the present invention resides in the fact that it has now become possible to perform the epoxidation reaction of step (e) continuously at a higher temperature than was previously possible in a commercial unit. It has now become possible to contact the hydroperoxide and the alkene continuously with the catalyst at a temperature of more than 85 °C. This temperature is the temperature of the mixture of hydroperoxide and the alkene when contacted with the catalyst for the first time. Sometimes, it is even possible to increase the temperature to at least 90 °C. The temperature will generally be at most 115°C, more specifically at most 100°C. However, the exact temperatures depend on the catalyst which is used as mentioned hereinabove.

A heterogeneous catalyst which suitably can be used in step (e) is a titanium containing catalyst. A preferred catalyst contains titanium on silica and/or silicate. A preferred catalyst is described in EP-A-345856. The reaction generally proceeds at moderate temperatures and pressures, in particular at temperatures in the range of from 0 to 200 °C, preferably in the range from 25 to 200 °C. The precise pressure is not critical as long as it suffices to maintain the reaction mixture as a liquid or as a mixture of vapour and liquid. Atmospheric pressure may be satisfactory. In general, pressures can be in the range of from 1 to 100 x 10⁵ N/m².

The alkylene oxide can be separated from the reaction mixture obtained in step (e) in any way known to be suitable to someone skilled in the art. The liquid reaction product may be worked up by fractional distillation, selective extraction and/or filtration. Any solvent, unreacted olefin and/or hydroperoxide may be recycled for further utilization. Preferably, in step (f) the alkylene oxide is separated by distillation from the reaction mixture.

The hydroxyl containing compounds obtained in the process can be dehydrated in the presence of a catalyst to obtain styrene and water. Processes which can be used for this step have been described in WO 99/42425 and WO 99/42426. However, any suitable process known to someone skilled in the art can in principle be used.

The invention is further illustrated by the following examples without limiting the scope of the invention to these particular embodiments.

### Comparative Example 1

In a reactor, air was blown through ethylbenzene. The product obtained contained ethylbenzene hydroperoxide. This product was contacted with a solution containing 0.5 %wt NaOH in water and mixed at a temperature of 60 °C. The weight ratio of product containing ethylbenzene hydroperoxide to NaOH containing solution was 4.5:1 (wt:wt). The neutralized mixture obtained was sent to a settling vessel where a neutralized hydrocarbonaceous phase containing ethylbenzene hydroperoxide was separated from an aqueous phase.

The neutralized hydrocarbonaceous phase containing ethylbenzene hydroperoxide was sent to a coalescer where further aqueous phase was removed. Subsequently, the neutralized hydrocarbonaceous phase containing ethylbenzene hydroperoxide, was washed by mixing with water, separating the mixture obtained in a settling vessel into an aqueous phase and a hydrocarbonaceous phase, subsequently separating the hydrocarbonaceous phase obtained from the settling vessel with the help of a coalescer. The hydrocarbonaceous phase obtained in the coalescer contains ethylbenzene hydroperoxide, ethylbenzene, water and contaminants. This hydrocarbonaceous phase is distilled. The distillate contains ethyl benzene, water and contaminants. The bottom product contains ethylbenzene hydroperoxide and ethylbenzene.

The ethylbenzene hydroperoxide product contained between 30 and 40 %wt of ethylbenzene hydroperoxide in ethylbenzene. A feed was obtained by mixing propene having a temperature of about 50 °C and the ethylbenzene hydroperoxide product having a temperature of about 97 °C in such amounts that the molar ratio of propene to ethylbenzene hydroperoxide was about 6. The feed obtained was heated in a heat-exchanger in which the heat was provided by steam having a temperature of about 160 °C and a pressure of about 5 bar (5 x 10⁵ N/m²).

At the start of operation, the feed was heated to about 95 °C. After several weeks of unchanged operation, the feed leaving the heat exchanger had a temperature of about 85 °C. This reduction in temperature attained shows substantial fouling of the heat exchanger.

### Example 1

The process set-up of Comparative Example 1 was changed in that only the propene having a temperature of about 50 °C was heated in the heat exchanger to a temperature of about 85 °C. The heated propene is subsequently combined with the ethylbenzene hydroperoxide product having a temperature of about 97 °C.

The combined heated propene and ethylbenzene hydroperoxide feed had a temperature of about 92 °C. It was found that this temperature could be maintained for more than a month.

## Claims

1. Process for the preparation of alkylene oxide which process comprises:
(a) oxidation of an organic compound to obtain a hydroperoxide containing stream,
(b) washing the hydroperoxide stream with a basic aqueous solution,
(c) washing the hydroperoxide stream of step (b) with water,
(d) optionally subjecting the hydroperoxide stream obtained in step (c) to distillation,
(e) contacting at least part of the hydroperoxide stream obtained in step (c) and/or (d) with alkene and a heterogeneous catalyst to obtain a reaction mixture containing a hydroxyl containing compound and alkylene oxide, and
(f) separating at least part of the alkylene oxide from the reaction mixture,
in which process the alkene added in step (e) has a temperature of from 60 to 120 °C while the temperature of the hydroperoxide stream contacted with the alkene is similar to the temperature of the hydroperoxide stream obtained in step (c) and/or (d).

2. Process according to claim 1, wherein the alkene is propene and the alkylene oxide is propylene oxide.

3. Process according to claim 1 or 2, wherein the heterogeneous epoxidation catalyst is a titanium containing catalyst.

4. Process according to any one of claims 1 to 3, wherein the alkene is heated in a heat exchanger before addition in step (e).

5. Process according to any one of claims 1 to 4, wherein the temperature of the alkene added in step (e) is of from 70 to 110 °C before contact with the hydroperoxide stream.

6. Process according to any one of claims 1 to 5, wherein the temperature of the hydroperoxide stream is of from 80 to 110 °C before contact with the alkene.

7. Process according to any one of claims 1 to 6, in which process in step (f) the alkylene oxide is separated from the reaction mixture by distillation.

## Patentansprüche

1. Verfahren zur Herstellung von Alkylenoxid, welches Verfahren umfaßt:
(a) die Oxidation einer organischen Verbindung, um einen Hydroperoxid enthaltenden Strom zu erhalten,
(b) das Waschen des Hydroperoxidstroms mit einer basischen wässerigen Lösung,
(c) das Waschen des Hydroperoxidstroms aus Schritt (b) mit Wasser,
(d) gegebenenfalls das Unterwerfen des in Schritt (c) erhaltenen Hydroperoxidstroms unter eine Destillation,
(e) das Inkontaktbringen von wenigstens einem Teil des in Schritt (c) und/oder (d) erhaltenen Hydroperoxidstroms mit Alken und einem heterogenen Katalysator, um eine ein Hydroxyl enthaltende Verbindung und Alkylenoxid enthaltende Reaktionsmischung zu erhalten, und
(f) das Abtrennen von wenigstens einem Teil des Alkylenoxids aus dem Reaktionsgemisch,
in welchem Verfahren das im Schritt (e) zugesetzte Alken eine Temperatur von 60 bis 120° C aufweist, während die Temperatur des mit dem Alken in Kontakt gebrachten Hydroperoxidstroms ähnlich der Temperatur des Hydroperoxidstroms ist, welcher im Schritt (c) und/oder (d) erhalten wird.

2. Verfahren nach Anspruch 1, worin das Alken Propen ist und das Alkylenoxid Propylenoxid ist.

3. Verfahren nach Anspruch 1 oder 2, worin der heterogene Epoxidierungskatalysator ein Titan enthaltender Katalysator ist.

4. Verfahren nach einem der Ansprüche 1 bis 3, worin das Alken vor der Zugabe im Schritt (e) in einem Wärmetauscher erwärmt wird.

5. Verfahren nach einem der Ansprüche 1 bis 4, worin die Temperatur des im Schritt (e) zugesetzten Alkens von 70 bis 110° C beträgt, bevor es mit dem Hydroperoxidstrom in Kontakt kommt.

6. Verfahren nach einem der Ansprüche 1 bis 5, worin die Temperatur des Hydroperoxidstroms von 80 bis 110°C beträgt, bevor er mit dem Alken in Kontakt kommt.

7. Verfahren nach einem der Ansprüche 1 bis 6, in welchem Verfahren im Schritt (f) das Alkylenoxid vom Reaktionsgemisch durch Destillation abgetrennt wird.

## Revendications

1. Procédé de préparation d'oxyde d'alkylène, lequel procédé comprend :
(a) l'oxydation d'un composé organique pour obtenir un courant contenant de l'hydroperoxyde,
(b) le lavage du courant à l'hydroperoxyde avec une solution aqueuse basique,
(c) le lavage du courant à l'hydroperoxyde de l'étape (b) avec de l'eau,
(d) éventuellement le traitement du courant à l'hydroperoxyde obtenu dans l'étape (c) en le soumettant à une distillation,
(e) la mise en contact d'au moins une partie du courant à l'hydroperoxyde obtenu dans l'étape (c) et/ou (d) avec un alcène et un catalyseur hétérogène pour obtenir un mélange de réaction contenant un composé contenant de l'hydroxyle et de l'oxyde d'alkylène, et
(f) la séparation d'au moins une partie de l'oxyde d'alkylène du mélange de réaction,
procédé dans lequel l'alcène ajouté dans l'étape (e) a une température de 60 à 120°C alors que la température du courant à l'hydroperoxyde mis en contact avec l'alcène est similaire à la température du courant à l'hydroperoxyde obtenu dans l'étape (c) et/ou (d).

2. Procédé suivant la revendication 1, dans lequel l'alcène est du propène et l'oxyde d'alkylène est de l'oxyde de propylène.

3. Procédé suivant l'une ou l'autre des revendications 1 et 2, dans lequel le catalyseur d'époxydation hétérogène est un catalyseur contenant du titane.

4. Procédé suivant l'une quelconque des revendications 1 à 3, dans lequel l'alcène est chauffé dans un échangeur de chaleur avant l'addition dans l'étape (e).

5. Procédé suivant l'une quelconque des revendications 1 à 4, dans lequel la température de l'alcène ajouté dans l'étape (e) est de 70 à 110°C avant la mise en contact avec le courant à l'hydroperoxyde.

6. Procédé suivant l'une quelconque des revendications 1 à 5, dans lequel la température du courant à l'hydroperoxyde est de 80 à 110°C avant la mise en contact avec l'alcène.

7. Procédé suivant l'une quelconque des revendications 1 à 6, procédé dans lequel dans l'étape (f) l'oxyde d'alkylène est séparé du mélange de réaction par distillation.
